# EUROPEAN PATENT SPECIFICATION

(11) **EP 0 704 195 B1**
(45) Date of publication and mention of the grant of the patent: **08.03.2000**
(21) Application number: 95402148.1
(22) Date of filing: 26.09.1995
(51) Int. Cl.: A61F 13/42

(54) **Menstrual detector**
Menstruationsdetektor
Détecteur de menstruation

(30) Priority: 27.09.1994 ES 9402454
(43) Date of publication of application: 03.04.1996
(73) Proprietor: Echeverria Salinas, Maria Teresa, 46022 Valencia (ES)
(72) Inventor: Echeverria Salinas, Maria Teresa, 46022 Valencia (ES)
(74) Representative: Joly, Jean-Jacques

(56) References cited:
- EP-A- 0 324 385
- US-A- 5 167 655
- DATABASE WPI Section Ch, Week 9308 Derwent Publications Ltd., London, GB; Class A14, AN 93-060959 & JP-A-05 007 618 ( NITTO DENKO CORP) , 19 January 1993

## Description

### TECHNICAL FIELD

This invention relates to a menstrual detector that discreetly warns the user of the arrival of her period.

### BACKGROUND OF THE INVENTION

One of the most common feminine hygiene products for controlling menstruation is the sanitary napkin. The appropriate time to use a sanitary napkin is difficult to determine, however, because of the irregularities that often attend menstrual cycles and, especially, the unpredictability of the precise moment that the flow will begin.

Although women have attempted to calculate the arrival of their periods by observing their physical symptoms, such efforts are tedious and the estimates may nevertheless be wrong. In the absence of any reliable means of predicting its onset, the period frequently arrives without the woman having taken the proper precautions, with the result of annoying and embarrassing stains on her clothes.

As a precautionary measure, a woman may use a smaller sanitary napkin during the days before she thinks that her period will arrive.In any case, she must be very watchful and, unfortunately, even so she cannot avoid the occasional flow leaking through to the skirt, pants, etc. Such risk increases for women who must be on their feet many hours, as is the case of teachers, shop assistants, nurses, etc.

### SUMMARY OF THE INVENTION

To provide a practical preventive solution for these drawbacks, a menstrual detector is proposed according to this invention.

The proposed detector basically comprises a sanitary napkin of reduced dimensions, formed by a thin layer of absorbent material, and within the absorbent material, a small cavity filled with a product, such as sodium thiosulfate or sodium hyposulfite, which can react to the temperature of the menstrual flow by turning cold upon coming into contact with and dissolving in the warm menstrual flow.

The cavity that contains the reactive product can extend as a thin layer throughout the interior of the absorbent material, or partially in only one area located anywhere in the absorbent material. Preferably, the cavity is situated in the central area of the absorbent material.

In this manner a completely effective detector is obtained, which, simply by producing a cold sensation, allows the user to be warned with total discretion of the arrival of her menstrual flow, so that she can take the proper precautions with sanitary napkins capable of greater absorption.

The reduced mass of material with which it is made makes the menstrual detector very inexpensive and easy to use. Furthermore, the detector is absolutely inoffensive, since the reactive material included inside is completely innocuous.

Because of all of the above reasons, the proposed detector is very useful for its intended purpose.

### BRIEF DESCRIPTION OF THE DRAWINGS

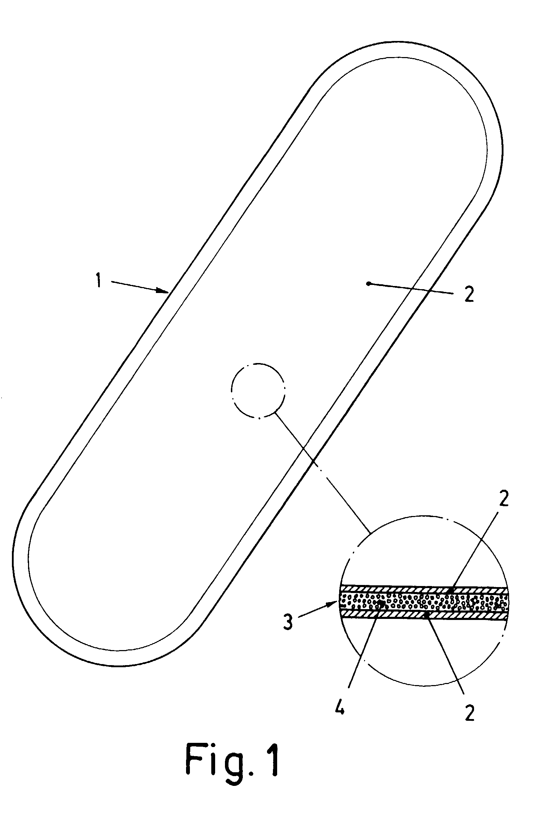

Figure 1 provides a perspective and a magnified cross-sectional view of a menstrual detector according to this invention.

### DETAILED DESCRIPTION OF THE INVENTION

To better understand the nature of this invention, in the attached drawings we show, by way of a merely illustrative, nonlimiting example, a preferred commercial application, to which we refer in our description. In the drawings:

Figure 1 represents a perspective view of the proposed detector, according to a nonlimiting example, with a magnified cross-sectional view of the area housing the internal reactive product.

The subject of the invention relates to a menstrual detector, intended to be used before menstruation to discreetly alert the user the moment her menstrual flow begins, so she may take the proper precautions to avoid the disagreeable and embarrassing situation of leakage through her clothes.

The proposed detector comprises a sanitary napkin of little thickness (1), formed by a thin layer of absorbent material (2), within whose interior a small cavity (3) is included and filled with a product (4) with specific reactive characteristics.

This product (4) preferably comprises sodium thiosulfate or sodium hyposulfite, which can respond by turning cold upon coming into contact with and dissolving in a hot liquid, such as a menstrual flow.

The sanitary napkin (1) can adopt any conventional shape or form, the only particularity being the reduced thickness of the absorbent material (2), so that its use will provide little discomfort.

The reduced thickness makes the sanitary napkin (1) optimally suited for use during the premenstrual period because the user can achieve secure protection against small flows with little discomfort. When the menstrual flow arrives, it dissolves product (4) contained in the internal cavity (3). The product (4) reacts by producing a cold sensation, so that the user becomes aware of the situation in an intimate and totally discrete manner, and can adopt appropriate measures to avoid embarrassing leakage by using more absorbent sanitary napkins.

Thus, sanitary napkin (1) is perfectly tailored to be used as a hygienic and protective shield during the premenstrual cycle, with the purpose of avoiding embarrassing situations. It is especially ideal for use on trips and in certain jobs which require standing and facing an audience, such as, for example, schoolteaching, etc.

The cavity (3) that contains the reactive product (4) can extend as a thin layer throughout the interior of the sanitary napkin (1), or partially in only one area located anywhere in the sanitary napkin (1). Preferably, the cavity (3) is situated in the central area, since this is where the flow first arrives and, hence, where its presence can be most rapidly detected. In any case, the presence of reactive product (4) will not be harmful to the user, since the substance employed is totally innocuous.

The nature of this invention being thus described, it will be obvious that the same may be varied in many ways. For instance, changes in the shape, material and structure may be introduced into the whole or constituent parts of the menstrual detector. Such modifications are intended to be included within the scope of the following claims.

## Claims

1. A menstrual detector comprising a sanitary napkin (1) of reduced dimensions, formed by a thin layer of absorbent material (2), and within the interior of the absorbent material a small cavity (3) containing a temperature-sensitive reactive chemical product (4) that can respond by turning cold when it comes into contact with and dissolves in a menstrual flow.

2. The menstrual detector of claim 1, wherein the reactive product (4) employed comprises sodium thiosulfate or sodium hyposulfite.

3. The menstrual detector of claim 1 or 2, wherein the cavity (3) containing the reactive product (4) extends as a thin layer throughout the interior of the absorbent material (2).

4. The menstrual detector of claim 1 or 2, wherein the cavity (3) affects only a partial area of the absorbent material (2).

5. The menstrual detector of claim 4, wherein the cavity (3) is located in the middle part of the absorbent material (2).

6. The menstrual detector of claim 2, wherein the reactive product (4) employed comprises sodium thiosulfate.

7. The menstrual detector of claim 2, wherein the reactive product (4) employed comprises sodium hyposulfite.

## Patentansprüche

1. Menstruationsdetektor, umfassend eine Damenbinde (1) mit reduzierten Abmessungen, die aus einer dünnen Lage absorbierenden Materials (2) gebildet ist, wobei im Inneren des absorbierenden Materials ein kleiner Hohlraum (3) vorhanden ist, der ein temperaturempfindliches, reaktionsfähiges, chemisches Produkt (4) enthält, das mit Kaltwerden reagieren kann, wenn es mit Menstruationsfluß in Berührung gelangt und sich darin auflöst.

2. Menstruationsdetektor nach Anspruch 1,
wobei das verwendete reaktionsfähige Produkt (4) Natriumthiosulfat oder Natriumhyposulfit aufweist.

3. Menstruationsdetektor nach Anspruch 1 oder 2,
wobei der das reaktionsfähige Produkt (4) enthaltende Hohlraum (3) sich als dünne Schicht durch das gesamte Innere des absorbierenden Materials (2) erstreckt.

4. Menstruationsdetektor nach Anspruch 1 oder 2,
wobei sich der Hohlraum (3) nur über einen Teilbereich des absorbierenden Materials (2) erstreckt.

5. Menstruationsdetektor nach Anspruch 4,
wobei sich der Hohlraum (4) in dem Mittelteil des absorbierenden Materials (2) befindet.

6. Menstruationsdetektor nach Anspruch 2,
wobei das verwendete reaktionsfähige Produkt (4) Natriumthiosulfat aufweist.

7. Menstruationsdetektor nach Anspruch 2,
wobei das verwendete reaktionsfähige Produkt (4) Natriumhyposulfit aufweist.

## Revendications

1. Détecteur de menstruations comprenant une serviette hygiénique (1) de dimensions réduites, constituée d'une mince couche d'un matériau absorbant (2) et à l'intérieur du matériau absorbant d'une petite cavité (3) contenant un produit chimique réactif sensible à la température (4), lequel peut répondre en devenant froid lorsqu'il est en contact avec un écoulement menstruel et qu'il se dissout dans un écoulement menstruel.

2. Détecteur de menstruations selon la revendication 1, dans lequel le produit réactif (4) utilisé comprend du thiosulfate de sodium ou de l'hyposulfite de sodium.

3. Détecteur de menstruations selon la revendication 1 ou 2, dans lequel la cavité (3) contenant le produit réactif (4) s'étend comme une mince couche d'un bout à l'autre à l'intérieur du matériau absorbant (2).

4. Détecteur de menstruations selon la revendication 1 ou 2, lequel la cavité (3) ne concerne qu'une zone partielle du matériau absorbant (2).

5. Détecteur de menstruations selon la revendication 4, dans lequel la cavité (3) est disposée dans la partie médiane du matériau absorbant (2).

6. Détecteur de menstruations selon la revendication 2, dans lequel le produit réactif (4) utilisé comprend du thiosulfate de sodium.

7. Détecteur de menstruations selon la revendication 2, dans lequel le produit réactif (4) utilisé comprend de l'hyposulfite de sodium.
